# EUROPEAN PATENT SPECIFICATION

(11) **EP 4 440 708 B1**
(45) Date of publication and mention of the grant of the patent: **24.06.2026**
(21) Application number: 22822426.7
(22) Date of filing: 25.11.2022
(51) Int. Cl.: A61Q 19/10, A61Q 5/02, A61K 8/02, A61K 8/20, A61K 8/46, A61K 8/44, A61K 8/368

(54) **HYDRATABLE CONCENTRATED SURFACTANT COMPOSITIONS**
HYDRATISIERBARE KONZENTRIERTE TENSIDZUSAMMENSETZUNGEN
COMPOSITIONS TENSIOACTIVES CONCENTRÉES HYDRATABLES

(30) Priority: 29.11.2021 EP 21211171
(43) Date of publication of application: 09.10.2024
(73) Proprietor: Unilever IP Holdings B.V., 6708 WH Wageningen (NL); Unilever Global IP Limited, Wirral, Merseyside CH62 4ZD (GB)
(72) Inventor: WEBB, Nicholas, 6708 WH Wageningen (NL); ZDRAVKOVA, Aneliya Nikolova, 6708 WH Wageningen (NL)
(74) Representative: Unilever Patent Group
(86) International application number: PCT/EP2022/083297
(87) International publication number: WO 2023/094600

(56) References cited:
- WO-A1-94/16680
- CA-A1- 2 135 641

## Description

### Field of the Invention

The present invention is directed to a hydratable concentrated surfactant cleansing composition and a method of making an end use composition by diluting with water. This invention has particular application in the field of personal care, particularly hair care.

### Background and Prior Art

Liquid based cleansing compositions, such as shampoos and body washes, are common and enjoyed by many consumers. Such compositions typically have water as the predominant ingredient, and they are often sold in plastic bottles or tubes. The compositions are conventionally formulated to have a viscosity that is customary for consumer use and easy for evacuation from the package they are sold in.

It is often publicised that the world's oceans will soon contain more plastic than fish.

Given environmental concerns and the desire for consumers and conscious companies to do more for the planet, there is a strong desire to use less plastic when selling products, including consumer products. In view of this, efforts have been made to sell product in concentrate form, and therefore, ship product that comprises less water, in smaller packages.

Surfactant containing cleansing products, in the form of concentrates are known in the prior art.

WO 19/205086 (Beiersdorf) describes a hair cleansing composition comprising anionic and amphoteric surfactants, a natural oil, an ethoxylated hydrogenated castor oil and a thickening agent.

US 2009/0208434 A (Symrise) discloses 3-(4-hydroxy-3-methoxyphenyl)-1-(4-hydroxyphenyl) -1-propanone and its use in cosmetic and pharmaceutical preparations. A concentrated body wash and shampoo are exemplified.

US 2009/0208434 A (Symrise) discloses 3-(4-hydroxy-3-methoxyphenyl)-1-(4-hydroxyphenyl) -1-propanone and its use in cosmetic and pharmaceutical preparations. A concentrated body wash and shampoo are exemplified.

WO 1994/16680 (Unilever) discloses an aqueous dilution-thickening concentrated personal washing compositions comprising: a) 20-60 wt % surfactant other than soap or primary alcohol sulphate, and b) 1-20 wt % electrolyte, in a lamellar phase.

WO10149425 (Unilever) discloses a concentrated shampoo composition comprising from 27 to 70 wt % cleansing surfactant, a conditioning gel phase, a short chain diol and an oil, the gel phase comprising fatty material, a gel network anionic surfactant and a cationic surfactant; wherein the condition gel network has no overall charge or is anionic.

WO 2020/044235 (Lonza) discloses a high-concentrate flowable liquid anionic surfactant composition comprising (a) an anionic surfactant solution comprising an alkyl ether sulfate, (b) a viscosity modifier comprising an aromatic alcohol, a linear or branched polyglyceryl ester, alkyl glyceryl ether or combinations thereof, and (c) an aqueous solvent wherein upon dilution with an aqueous solvent, the composition remains flowable.

US2021/0128447 (Unilever) discloses a sulphate free composition comprising, in an aqueous continuous phase: a total amount of anionic surfactant, amphoteric surfactant and zwitterionic surfactant consisting of: (i) 3 to 13 wt %, by weight of the total composition at 100 percent activity, of an alpha olefin sulfonate anionic surfactant of a defined formula (I): R¹-CH-CH-CH₂-SO₃⁻M⁺ (I) in which R¹ is selected from linear or branched alkyl groups having from 11 to 13 carbon atoms and mixtures thereof; and M is a solubilizing cation; (ii) 1 to 6 %, by weight of an amphoteric or zwitterionic surfactant, selected from an alkyl betaine of general formula (II): R²-N⁺(CH₃)₂-CH₂-COO⁻M⁺ (II) wherein R²=C12 (Lauryl) or Coco derived; an alkyl hydroxy sultaine of general formula (III), R³-N⁺(CH₃)₂-CH₂-CH(OH)-CH₂-SO₃⁻M⁺ (III), wherein R³=C12 (Lauryl) or Coco derived; an alkyl aminopropyl hydroxy sultaine of general formula (IV), R⁴-CO-NH-(CH₂)₃-N⁺(CH₃)₂-CH₂-CH(OH)-CH₂-SO₃⁻M⁺ (IV) wherein R⁴=C12 (Lauryl) or Coco derived; an alkyl amphoacetate of general formula (V), R⁵-CO-NH-(CH₂)₂-N(CH₂-CH₂-OH)(CH₂-COO⁻M⁺) (V), wherein R⁵=C12 (Lauryl) or Coco derived; and mixtures thereof; (iii) from 0.05 to 0.9 wt percent of a cationic conditioning polymer; (iv) from 0.05 to 10 wt percent of a silicone emulsion; (v) a cationic deposition polymer; (vi) an inorganic electrolyte; and (vii) water; in which the weight ratio of (i) to (ii) ranges from 1:1 to 6:1 and the pH of the composition is from 3 to 6.5. Agreeable viscosity and foaming properties are achieved by the exemplified standard rinse-off compositions which comprise silicone and sodium benzoate.

WO18002557A1 uses high concentrations (35-60 percent) of surfactant mixtures including sulfate free anionic, amphoteric and non-ionic that access the lamellar phase region to control the rheology. These formulas have no added salt. Although high surfactant concentrations in lamellar phase solve the rheology build problem, they may not favour mild benefits or have the in-use application and sensory characteristics of an isotropic shampoo, such as transparent appearance and flash foam.

Concentrates may be diluted at point of use to form a liquid product that is then usable in the conventional way.

A difficulty encountered with such concentrates is that consumers often do not like adding additional water to the concentrate and having to convert, for example by stirring, the concentrate into a usable end product. As to the hydrated product, common complaints include that the process is time consuming and the resulting product is not homogeneous, and has undesirable viscosity, after adding water.

Indeed, we have found that such concentrated compositions take extended time to dilute properly, prior to use by the consumer and some consumers do not allow enough time for full dilution to take place. It is, therefore, desirable to provide a concentrate that dilutes quickly for easier and more efficient use.

The properties of the diluted concentrate are also important. Consumers strongly prefer thick liquids because they associate thickness with quality, good spreadability and pourability. Ideally, the diluted product of a concentrate would have similar viscosity to a standard format personal care product, such as a shampoo.

Despite the prior art there remains the need for improved concentrated shampoo compositions.

Typically, the phase structure of a regular, non-concentrated shampoo composition is in the isotropic phase. However, in providing a concentrated shampoo, as the surfactant concentration is increased, the typical rheology moves from the isotropic phase to the hexagonal phase. The hexagonal phase provides a composition which is too viscous to be suitable for the typical consumer and can only be diluted very slowly.

We have now surprisingly found that a highly concentrated composition, in the hexagonal phase, can be modified into a lamellar crystal phase by the use of sodium benzoate. This composition is capable of transforming rapidly to an isotropic phase upon dilution. Furthermore, in combination with a high salt content, a thick end dilute can be achieved.

The composition can be used as a concentrate in small volumes and diluted at point of use. Thus, it is ready to use, in dilute form, at the time that it is required. It can be diluted with water in refill packaging to ensure a reduction in plastic waste.

### Definition of the Invention

In a first aspect, the invention provides a hydratable concentrated surfactant composition comprising:
a) from 30 to 50 wt % of anionic surfactant selected from sodium laureth ether sulphate, sodium pareth ether sulphate and mixtures thereof, by weight based on the total weight of the composition;
b) from 4 to 12 wt %, of a co-surfactant, which is selected from a betaine, cocamide monoethanolamide and mixtures thereof, based on the total weight of the hydratable concentrated surfactant composition;
c) from 0.2 to 3 wt % of a phase modifier, by weight of the total composition, which is sodium benzoate;
d) from 2 to 7 wt % of a viscosity modifier, by weight of the total composition, which is an inorganic electrolyte;
e) from 10 to 60 % by weight water by weight of the total composition;

wherein the surfactants are in a lamellar phase; and
wherein the hydratable concentrated surfactant composition has a pH of from 3 to 6; and
wherein the hydratable concentrated surfactant composition has a viscosity of from 5,000 to 100,000 mPas⁻¹ (cps), when measured at 30°C on a Brookfield DV2T using a Spindle RV-05 at 20 rpm for 60 seconds on a Helipath stand; and
wherein the weight ratio of a) the anionic surfactant to b) the co- surfactant is from 3:1 to 10:1; and
wherein the weight ratio of sodium benzoate (c) to total surfactant (a) + (b) is 1:5 - 1: 75, preferably from 1:10 - 1:65, more preferably 1:15 - 1:50.

An end use composition can be made by hydrating the hydratable concentrated surfactant composition of the first aspect by dilution with water.

Preferably, the end use composition has a weight ratio of hydratable concentrated surfactant composition to water of from 1:1 to 1:6, more preferably 1:1 to 1:5.

The hydratable concentrated surfactant composition has a viscosity of from 5,000 to 100,000 mPas⁻¹ (cps), preferably from 7,000 to 90,000, more preferably from 8,000 to 75,000 mPas⁻¹ (cps).

Upon dilution the viscosity decreases, thus resulting in an end use composition having a viscosity of from 100 to 7,000 mPas⁻¹ (cps), preferably from 1000 to 6,000 mPas⁻¹ (cps), more preferably from 2000 to 5000 mPas⁻¹ (cps) when measured at 30°C on a Brookfield DV2T using a Spindle RV-05 at 20 rpm for 60 seconds on a Helipath stand.

In a second aspect, the present invention provides a method of preparing an end use composition, the method comprising the step of diluting a hydratable concentrated surfactant composition of the first aspect with water. Preferably, the method comprises a step of applying moderate shear such as shaking or stirring to the mixture of hydratable composition and water to yield the end use composition in less than 10 minutes, preferably less than 5 minutes, more preferably in less than 3 minutes, still more preferably in less than 2 minutes, even more preferably in less than 1 minute and most preferably in less than 30 seconds.

Preferably, the hydratable concentrated surfactant composition is diluted at a hydratable concentrated surfactant composition to water weight ratio of from 1:1 to 1:6, more preferably from 1:1 to 1:5. Preferably, the water has a temperature of from 10 to 50 degrees Celsius.

The diluted product is formed before it is used. Preferably, the composition is diluted before it is applied, for example to hair or skin, in a suitable container. In a preferred embodiment, the container is a refill package.

### Detailed Description of the Invention

When making the hydratable concentrated surfactant composition of the present invention, the desired ingredients may be mixed with conventional apparatus under moderate shear and atmospheric conditions, with temperature being from 35 to 80°C.

Water is added to the hydratable concentrated surfactant composition to produce the end use composition. Moderate shear such as shaking (or stirring) in a container will yield the end use composition in less than 5 minutes, preferably in less than 3 minutes, and most preferably, in less than 2 minutes. In an embodiment of the invention, end use composition is made in less than 1 minute, even preferably, less than 30 seconds. The end use composition is then ready to use.

In the present invention, the hydratable concentrated surfactant composition should be formulated such that upon dilution, the desired component/ingredient levels (such as sulfate levels) in the end use composition are attained.

The hydratable concentrated surfactant composition of the present invention will have a starting viscosity that is higher than the final viscosity after water is added and the resulting end use composition is made.

The hydratable concentrated surfactant composition and end use composition typically have a pH from 3 to 6.

The end use composition is made by combining water and the hydratable concentrated surfactant composition and mixing (with moderate shear like stirring, preferably shaking) to produce the end use composition.

The compositions of the invention are cosmetic and non-therapeutic.

The end use composition can be a personal care cleaning composition and is preferably a shampoo, make-up wash, facial wash, hand wash or personal care liquid body wash, more preferably a shampoo or a body wash, most preferably a shampoo.

The end use composition is one suitable to be wiped or washed off, and preferably, washed off with water.

In an embodiment of the invention, the end use shampoo composition can preferably have a viscosity from 100 to 6,000.

Viscosity, unless noted otherwise, is measured at 30°C on a Brookfield DV2T using a Spindle RV-05 at 20 rpm for 60 seconds on a Helipath stand.

Preferably, the hydratable concentrated surfactant compositions of the invention are free from silicone. In the context of the invention, by free from is meant having less than 0.4 weight %, more preferably less than 0.1 weight %, even more preferably less than 0.05 weight %, still more preferably less than 0.001 weight %, yet preferably less than 0.0001 weight %, and most preferably 0 weight % of silicone by weight of the total composition.

### The anionic surfactant

The hydratable concentrated surfactant composition of the invention includes an anionic surfactant, selected from sodium laureth ether sulphate (SLES), sodium pareth ether sulphate (SPES) and mixtures thereof.

In a typical hydratable concentrated surfactant composition of the invention the level of anionic surfactant will generally range from 30 to 50 %, preferably from 40 to 50 (by weight based on the total weight of the composition and 100 % activity).

### The co-surfactant

The hydratable concentrated surfactant composition of the invention includes a co-surfactant, which is selected from a betaine, cocamide monoethanolamide (CMEA) and mixtures thereof.

The level of co-surfactant is from 4 to 12 wt %, preferably 4 to 10 wt %, most preferably from 4 to 7.5 wt %, by weight based on the total weight of the hydratable concentrated surfactant composition and based on 100 % active level.

Betaines that are suitable for use in the present invention can be represented by the general formula:

R¹⁰[C(O)NH-(CH₂)_{y}]_{z} -N⁺(R¹¹)(R¹²) CH₂CO₂⁻ (IV)

where R¹⁰ is C6 to C30, more particularly C6 to C24 alkyl, z is 0 or 1, R¹¹ and R¹² are independently alkyl, hydroxyalkyl or carboxyalkyl of 1 to 3 carbon atoms, and y is 2 or 3; and salts thereof. In one embodiment, at least half of the groups R¹⁰ are C8 to C18 alkyl. In another embodiment, at least half of the groups R¹⁰ are C 10 to C14 alkyl. R¹⁰ may be saturated or unsaturated. In one embodiment, R¹⁰ is derived from coconut oil or palm kernel oil. In one embodiment R¹¹ and R¹² are methyl.

The formula (IV) betaines include the simple betaines:

R¹⁰ -N⁺(R¹¹)(R¹²) CH₂CO₂⁻ (IVa)

where R¹⁰, R¹¹, and R¹² are as described above, and the amidobetaines:

R¹⁰C(O)NH-(CH₂)_{y} -N⁺(R¹¹)(R¹²) CH₂CO₃⁻ (IVb)

where R¹⁰, R¹¹, R¹², and y are as described above.

Preferred betaines are oleyl betaine, caprylamidopropyl betaine, lauramidopropyl betaine, isostearylamidopropyl betaine, and cocoamidopropyl betaine and mixtures thereof. Most preferably, the co-surfactant is cocamidopropyl betaine.

Most preferably, the cosurfactant is selected from cocamidopropylbetaine (CAPB), cocamide monoethanolamide (CMEA) and mixtures thereof.

The weight ratio of a) the anionic surfactant to b) the co- surfactant is from 3:1 to 10:1, preferably from 3.5: 1 to 9:1, most preferably from 3.6:1 to 8.7:1.

### The Phase Modifier

The hydratable concentrated surfactant composition of the invention comprises a phase modifier, which moves the hexagonal phase to a lamellar phase, making it easier to dilute quickly.

The phase modifier is sodium benzoate.

The phase modifier is present in an amount of from 0.2 wt % to 3 wt % , preferably 0.5 wt % to 3 wt %, by weight of the total composition.

The weight ratio of sodium benzoate (c) to total surfactant (a) + (b) is 1:5 to 1: 75, preferably from 1:10 to 1:65, most preferably 1:15 to 1:50.

### The Viscosity Modifer

The hydratable concentrated surfactant composition of the invention comprises a viscosity modifier, which is an inorganic electrolyte, to increase the viscosity of the dilute end composition. Suitable inorganic electrolytes for use in the invention include metal chlorides (such as sodium chloride, potassium chloride, calcium chloride, magnesium chloride, zinc chloride, ferric chloride and aluminum chloride) and metal sulphates (such as sodium sulphate and magnesium sulphate) and mixtures thereof. The inorganic electrolyte is used to provide viscosity to the dilute end composition. Without it, the end composition is unacceptably thin to the consumer.

Mixtures of any of the above described materials may also be suitable.

Examples of preferred inorganic electrolytes for use in the invention include sodium chloride, potassium chloride, magnesium sulphate and mixtures thereof.

The level of inorganic electrolyte in compositions of the invention ranges from 2 to 7 wt%, preferably from 2 to 5 wt%, most preferably from 2 to 3 wt% (by total weight of the composition).

### Water

Water makes up from 10 to 60 wt %, preferably from 20 to 60 wt % by weight based on total weight of the composition.

The pH of the hydratable composition and end use composition is from 3 to 6, preferably from 3.5 to 5.5, more preferably from 3.5 to 5, most preferably from 3.8 to 4.8. Adjusters suitable to modify or buffer the pH may be used. Such pH adjusters include triethylamine, NaOH, KOH, H₂SO₄, HCl, citric acid or mixtures thereof. The pH adjusters are added at amounts to yield the desired final pH. The pH values may be assessed with commercial instrumentation such as a pH meter made commercially available from Thermo Scientific^{®}.

### Other Ingredients

The composition according to the invention may comprise any of a number of ingredients which are common to hair conditioning compositions.

Other ingredients may include preservatives, conditioning enhancing polymers, colouring agents, polyols such as glycerine and polypropylene glycol, chelating agents, antioxidants such as vitamin E acetate, fragrances, antimicrobials and sunscreens. Each of these ingredients will be present in an amount effective to accomplish its purpose. Generally these optional ingredients are included individually at a level of up to about 5% by weight of the total composition.

Preferably, the further ingredients include fragrances, preservatives and conditioning enhancing polymers.

### Preservatives

Preservatives are used in the hydratable concentrated surfactant composition and end use composition to protect against the growth of potentially harmful microorganisms.

Preferably, the preservative comprises an alkali metal salt of benzoic acid, preferably sodium benzoate and a metal ion sequestrant, which is preferably ethylenediaminetetraacetic acid (EDTA).

Preferably, the concentrated composition of the invention comprises EDTA.

### Optional cationic polymers

Cationic polymers are preferred ingredients in compositions of the invention for enhancing conditioning performance.

Suitable cationic polymers may be homopolymers which are cationically substituted or may be formed from two or more types of monomers. The weight average (M_{w}) molecular weight of the polymers will generally be between 100 000 and 3 million daltons. The polymers will have cationic nitrogen containing groups such as quaternary ammonium or protonated amino groups, or a mixture thereof. If the molecular weight of the polymer is too low, then the conditioning effect is poor. If too high, then there may be problems of high extensional viscosity leading to stringiness of the composition when it is poured.

The cationic nitrogen-containing group will generally be present as a substituent on a fraction of the total monomer units of the cationic polymer. Thus when the polymer is not a homopolymer it can contain spacer non-cationic monomer units. Such polymers are described in the CTFA Cosmetic Ingredient Directory, 3rd edition. The ratio of the cationic to non-cationic monomer units is selected to give polymers having a cationic charge density in the required range, which is generally from 0.2 to 3.0 meq/gm. The cationic charge density of the polymer is suitably determined via the Kjeldahl method as described in the US Pharmacopoeia under chemical tests for nitrogen determination.

Suitable cationic polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general, secondary and tertiary amines, especially tertiary, are preferred.

Amine substituted vinyl monomers and amines can be polymerised in the amine form and then converted to ammonium by quaternization.

The cationic polymers can comprise mixtures of monomer units derived from amine- and/or quaternary ammonium-substituted monomer and/or compatible spacer monomers. Suitable (non-limiting examples of) cationic polymers include:
- cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively;
- mineral acid salts of amino-alkyl esters of homo-and co-polymers of unsaturated carboxylic acids having from 3 to 5 carbon atoms, (as described in U.S. Patent 4,009,256);
- cationic polyacrylamides(as described in WO95/22311).

Other cationic polymers that can be used include cationic polysaccharide polymers, such as cationic cellulose derivatives, cationic starch derivatives, and cationic guar gum derivatives. Cationic polysaccharide polymers suitable for use in compositions for use in the invention include monomers of the formula:

A-O-[R-N⁺(R¹)(R²)(R³)X⁻],

wherein: A is an anhydroglucose residual group, such as a starch or cellulose anhydroglucose residual. R is an alkylene, oxyalkylene, polyoxyalkylene, or hydroxyalkylene group, or combination thereof. R¹, R² and R³ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in R¹, R² and R³) is preferably about 20 or less, and X is an anionic counterion.

Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from the Amerchol Corporation, for instance under the tradename Polymer LM-200.

Other suitable cationic polysaccharide polymers include quaternary nitrogen-containing cellulose ethers (e.g. as described in U.S. Patent 3,962,418), and copolymers of etherified cellulose and starch (e.g. as described in U.S. Patent 3,958,581). Examples of such materials include the polymer LR and JR series from Dow, generally referred to in the industry (CTFA) as Polyquaternium 10.

A particularly suitable type of cationic polysaccharide polymer that can be used is a cationic guar gum derivative, such as guar hydroxypropyltrimethylammonium chloride (commercially available from Rhodia in their JAGUAR trademark series). Examples of such materials are JAGUAR C13S, JAGUAR C14 and JAGUAR C17.

Mixtures of any of the above cationic polymers may be used.

Cationic polymer will generally be present in a shampoo composition for use in the invention at levels of from 0.01 to 5%, preferably from 0.02 to 1%, more preferably from 0.05 to 0.8% by total weight of cationic polymer based on the total weight of the composition.

The Examples provided are to facilitate an understanding of the invention.

### Examples

### Example 1: Compositions 1 to 4 in accordance with the invention and comparative

### Compositions A to J

Concentrated shampoo compositions 1 - 4 were prepared in accordance with the invention. Comparative concentrated shampoo compositions A - J were also prepared. The compositions of Compositions 1- 2, in accordance with the invention, and Comparative compositions A to E are shown in Table 1 below.

The compositions of Compositions 3-4, in accordance with the invention, and Comparative compositions F to J are shown in Table 2 below.

**Table 1: Composition of Compositions 1- 2 in accordance with the invention and Comparative compositions A to E, also showing surfactant phase and viscosities in concentrate and dilute forms. Ratio (a): (b) = 7.5:1**

| Ingredient | Activity (wt %) of raw material | A | B | C | D | E | 1 | 2 |
|---|---|---|---|---|---|---|---|---|
| | | Wt % inclusion level as 100% | | | | | | |
| Water | to 100 | to 100 | to 100 | to 100 | to 100 | to 100 | to 100 | to 100 |
| Cocamidopropyl Betaine | 85 | 5.4 | 5.4 | 5.4 | 5.4 | 5.4 | 5.4 | 5.4 |
| Sodium Laureth Sulphate | 70 | 40.6 | 40.6 | 40.6 | 40.6 | 40.6 | 40.6 | 40.6 |
| Sodium Benzoate | 100 | 0 | 0.5 | 1.5 | 2.0 | 3.0 | 1.5 | 1.5 |
| Citric Acid | 100 | 3.4 | 3.4 | 3.4 | 3.4 | 3.4 | 3.4 | 3.4 |
| Sodium chloride | - | 0 | 0 | 0 | 0 | 0 | 3 | 5 |
| Surfactant phase (Hexagonal (H) or Lamellar (L)) | - | H | H | L | L | L | L | L |
| Concentrate viscosity (cp) | | 87267 | 71,748 | 13109 | 7140 | 5556 | 24136 | 41594 |
| Dilution time | | >30 | >30 | <5 | <3 | <3 | <5 | <5 |
| Dilute viscosity (cPs) | | - | - | 6 | 6 | 9 | 200 | 2970 |

**Table 2: Composition of Compositions 3 & 4 in accordance with the invention and Comparative compositions F to J, also showing surfactant phase and viscosities in concentrate and dilute forms. Ratio (a): (b) = 6.7:1**

| Ingredient | Activity (wt %) of raw material | F | G | H | I | J | 3 | 4 |
|---|---|---|---|---|---|---|---|---|
| | | Wt % Inclusion level as 100% | | | | | | |
| Water | To 100 | to 100 | To 100 | to 100 | to 100 | To 100 | To 100 | To 100 |
| Cocamidopropyl Betaine | 85 | 6 | 6 | 6 | 6 | 6 | 6 | 6 |
| Sodium Laureth Sulphate | 70 | 40 | 40 | 40 | 40 | 40 | 40 | 40 |
| Sodium Benzoate | 100 | 0 | 0.5 | 1.5 | 2.0 | 3.0 | 1.5 | 1.5 |
| Citric Acid | 100 | 3.4 | 3.4 | 3.4 | 3.4 | 3.4 | 3.4 | 3.4 |
| Sodium chloride | - | 0 | 0 | 0 | 0 | 0 | 3 | 5 |
| Surfactant phase (Hexagonal (H) or Lamellar (L)) | - | H | H | L | L | L | L | L |
| Concentrate viscosity (cp) | | 61916 | 56721 | 9140 | 8458 | 7127 | 27334 | 40189 |
| Dilution time | | >30 | >30 | <5 | <3 | <3 | <5 | <5 |
| Dilute viscosity (mPas⁻¹ (cps)) | | - | - | 6 | 6 | 8 | 300 | 4390 |

All compositions had a pH in the range of 3.5 to 4.5.

Concentrated compositions 1-4 were soft, thick and flowable.

Comparative Examples A and B were harder and solid like.

Concentrated shampoo compositions 1- 4, and Comparative compositions A to J were prepared by the following method:
- Sodium benzoate was dissolved in hot water.
- Sodium laureth sulphate was added and mixed under constant shear.
- Cocamidopropyl betaine was added and mixed until homogeneous.
- Finally, citric acid was added to adjust pH.

Viscosities of Compositions 1-4 and comparative Compositions A to J were measured at 30°C on a Brookfield DV2T using a Spindle RV-05 at 20 rpm for 60 seconds on a Helipath stand.

The surfactant phase was determined using the following method:
A polarising optical microscope (Zeiss Axio M1m Light Microscope) was used to obtain images of the compositions. A small amount of composition was placed on a microscope glass slide with a cover slip on top. In transmittance mode, a 20X magnification objective was used to capture an image under cross polarised light.

### Example 2: Dilution of concentrated Compositions 1-4 and Comparative compositions A to J to obtain an end use diluted product

Concentrated compositions 1- 4 and A to J were diluted for use as dilute shampoos are follows:
In the following method, the amounts of concentrate to water that were used to make 500g of the end use product was 1 part concentrate to 3 parts water.
1. Weigh out the required amount of concentrate formulation into a clear container.
2. Boil water and cool down until temperature reaches 50°C.
3. Add the correct amount of cooled boiled water to the container.
4. Seal container and shake for 30 seconds.
5. Leave to stand and evaluate undissolved parts every minute.
6. At 5 minutes evaluate again and shake container again for 30 seconds if required.
7. Dissolution time was measured.

Dilution times are given in Tables 1 and 2 above.

It will be seen that the compositions of the invention diluted significantly faster than the comparative examples. The viscosities of the dilute compositions were much greater for the compositions in accordance with the invention.

## Claims

1. A hydratable concentrated surfactant composition comprising:
a) from 30 to 50 wt % of anionic surfactant selected from sodium laureth ether sulphate, sodium pareth ether sulphate and mixtures thereof, by weight based on the total weight of the composition;
b) from 4 to 12 wt %, of a co-surfactant, which is selected from a betaine, cocamide monoethanolamide and mixtures thereof, based on the total weight of the hydratable concentrated surfactant composition;
c) from 0.2 to 3 wt % of a phase modifier, by weight of the total composition, which is sodium benzoate;
d) from 2 to 7 wt % of a viscosity modifier, by weight of the total composition, which is an inorganic electrolyte;
e) from 10 to 60 % by weight water by weight of the total composition;
o wherein the surfactants are in a lamellar phase; and
∘ wherein the hydratable concentrated surfactant composition has a pH of from 3 to 6; and
∘ wherein the hydratable concentrated surfactant composition has a viscosity of from 5,000 to 100,000 mPas⁻¹ (cps), when measured at 30°C on a Brookfield DV2T using a Spindle RV-05 at 20 rpm for 60 seconds on a Helipath stand; and
∘ wherein the weight ratio of a) the anionic surfactant to b) the co- surfactant is from 3:1 to 10:1,
∘ wherein the weight ratio of sodium benzoate (c) to total surfactant (a) + (b) is 1:5 - 1: 75, preferably from 1:10 - 1:65, more preferably 1:15 - 1:50.

2. A concentrated composition as claimed in claim 1, wherein the cosurfactant is selected from cocamidopropylbetaine, cocamide monoethanolamide and mixtures thereof.

3. A concentrated composition as claimed in any preceding claim, wherein the inorganic electrolyte is selected from metal chlorides, metal sulphates, and mixtures thereof.

4. A concentrated composition as claimed in any preceding claim, wherein the weight ratio of a) the anionic surfactant to b) the co- surfactant is from 3.5: 1 to 9:1.

5. A concentrated composition as claimed in any preceding claim, which further comprises ethylenediaminetetraacetic acid.

6. A concentrated composition as claimed in any preceding claim, which is free from silicone.

7. A method of preparing an end use composition, the method comprising the step of diluting a hydratable composition according to any one of claims 1 to 6 with water, prior to use.

8. A method as claimed in claim 7, wherein method comprises a step of applying shear such as shaking or stirring to the mixture of hydratable composition and water to yield the end use composition in less than 10 min.

9. A method as claimed in claim 7 or claim 8, wherein the composition is diluted in a container.

10. A method as claimed in claim 9, wherein the container is a refill package.

11. A method as claimed in claim 7 to 10, wherein the weight ratio of hydratable concentrated surfactant composition to water of from 1:1 to 1:6.

12. A method as claimed in any one of claims 7 to 11, wherein the end use composition is in the isotropic phase.

13. A method as claimed in any one of claims 7 to 12, wherein the end use composition has a viscosity of from 2000 to 10,000 mPas⁻¹ (cps) when measured at 30°C on a Brookfield DV2T using a Spindle RV-05 at 20 rpm for 60 seconds on a Helipath stand.

## Patentansprüche

1. Hydratisierbare konzentrierte Tensidzusammensetzung, umfassend:
a) 30 bis 50 Gew.-% anionisches Tensid, ausgewählt unter Natriumlaurethsulfat, Natriumparethsulfat und Mischungen davon, bezogen auf das Gesamtgewicht der Zusammensetzung;
b) 4 bis 12 Gew.-% eines Co-Tensids, das unter einem Betain, Cocamidmonoethanolamid und Mischungen davon ausgewählt ist, bezogen auf das Gesamtgewicht der hydratisierbaren konzentrierten Tensidzusammensetzung;
c) 0,2 bis 3 Gew.-% eines Phasenmodifikators, bezogen auf das Gesamtgewicht der Zusammensetzung, der Natriumbenzoat ist;
d) 2 bis 7 Gew.-% eines Viskositätsmodifikators, bezogen auf das Gesamtgewicht der Zusammensetzung, der ein anorganischer Elektrolyt ist;
e) 10 bis 60 Gew.-% Wasser, bezogen auf das Gesamtgewicht der Zusammensetzung;
wobei sich die Tenside in einer lamellaren Phase befinden; und
wobei die hydratisierbare konzentrierte Tensidzusammensetzung einen pH-Wert von 3 bis 6 aufweist; und
wobei die hydratisierbare konzentrierte Tensidzusammensetzung eine Viskosität von 5.000 bis 100.000 mPas⁻¹ (cps) aufweist, gemessen bei 30°C auf einem Brookfield DV2T unter Verwendung einer Spindel RV-05 bei 20 U/min für 60 Sekunden auf einem Helipath-Ständer; und
wobei das Gewichtsverhältnis von a) dem anionischen Tensid zu b) dem Co-Tensid 3:1 bis 10:1 beträgt,
wobei das Gewichtsverhältnis von Natriumbenzoat (c) zu Gesamt-Tensid (a) + (b) 1:5 bis 1:75, vorzugsweise 1:10 bis 1:65, bevorzugter 1:15 bis 1:50 beträgt.

2. Konzentrierte Zusammensetzung, wie im Anspruch 1 beansprucht, wobei das Co-Tensid unter Cocamidopropylbetain, Cocamidomonoethanolamid und Mischungen davon ausgewählt ist.

3. Konzentrierte Zusammensetzung, wie in einem vorhergehenden Anspruch beansprucht, wobei der anorganische Elektrolyt unter Metallchloriden, Metallsulfaten und Mischungen davon ausgewählt ist.

4. Konzentrierte Zusammensetzung, wie in einem vorhergehenden Anspruch beansprucht, wobei das Gewichtsverhältnis von a) dem anionischen Tensid zu b) dem Co-Tensid 3,5:1 bis 9:1 beträgt.

5. Konzentrierte Zusammensetzung, wie in einem vorhergehenden Anspruch beansprucht, die ferner Ethylendiamintetraessigsäure umfasst.

6. Konzentrierte Zusammensetzung, wie in einem vorhergehenden Anspruch beansprucht, die frei von Silikon ist.

7. Verfahren zur Herstellung einer Endverwendungszusammensetzung, wobei das Verfahren den Schritt des Verdünnens der hydratisierbaren Zusammensetzung nach irgendeinem der Ansprüche 1 bis 6 mit Wasser vor der Verwendung umfasst.

8. Verfahren, wie im Anspruch 7 beansprucht, wobei das Verfahren einen Schritt umfasst, bei dem auf die Mischung aus hydratisierbarer Zusammensetzung und Wasser eine Scherkraft, wie durch Schütteln oder Rühren, ausgeübt wird, um die Endverwendungszusammensetzung in weniger als 10 Minuten zu erhalten.

9. Verfahren, wie in Anspruch 7 oder Anspruch 8 beansprucht, wobei die Zusammensetzung in einem Behälter verdünnt wird.

10. Verfahren, wie im Anspruch 9 beansprucht, wobei der Behälter eine Nachfüllpackung ist.

11. Verfahren, wie im Anspruch 7 bis 10 beansprucht, wobei das Gewichtsverhältnis von hydratisierbarer konzentrierter Tensidzusammensetzung zu Wasser 1:1 bis 1:6 beträgt.

12. Verfahren, wie in einem der Ansprüche 7 bis 11 beansprucht, wobei sich die Endverwendungszusammensetzung in der isotropen Phase befindet.

13. Verfahren, wie in einem der Ansprüche 7 bis 12 beansprucht, wobei die Endverwendungszusammensetzung eine Viskosität von 2000 bis 10.000 mPas⁻¹ (cps) aufweist, gemessen bei 30°C auf einem Brookfield DV2T unter Verwendung einer Spindel RV-05 bei 20 U/min für 60 Sekunden auf einem Helipath-Stativ.

## Revendications

1. Composition de tensioactif concentrée hydratable comprenant :
a) 30 à 50 % en poids de tensioactif anionique choisi parmi le laureth-éthersulfate de sodium, le pareth-éthersulfate de sodium et leurs mélanges, en poids par rapport au poids total de la composition ;
b) 4 à 12 % en poids d'un co-tensioactif qui est choisi parmi une bétaïne, le coco-amidomonoéthanolamide et leurs mélanges, par rapport au poids total de la composition de tensioactif concentrée hydratable ;
c) 0,2 à 3 % en poids d'un modificateur de phase, par rapport au poids de la composition totale, qui est le benzoate de sodium ;
d) 2 à 7 % en poids d'un modificateur de viscosité, par rapport au poids de la composition totale, qui est un électrolyte inorganique ;
e) 10 à 60 % en poids d'eau, par rapport au poids de la composition totale ;
o dans laquelle les tensioactifs sont dans une phase lamellaire ; et
o dans laquelle la composition de tensioactif concentrée hydratable a un pH de 3 à 6 ; et
o dans laquelle la composition de tensioactif concentrée hydratable a une viscosité de 5 000 à 100 000 mPas⁻¹ (cps), quand elle est mesurée à 30 °C sur un dispositif Brookfield DV2T utilisant une broche RV-05 à 20 t/min pendant 60 secondes sur un support Helipath ; et
o dans laquelle le rapport en poids du tensioactif anionique a) au co-tensioactif b) est de 3/1 à 10/1 ;
o dans laquelle le rapport en poids du benzoate de sodium (c) au tensioactif total (a) + (b) est de 1/5 à 1/75, de préférence de 1/10 à 1/65, mieux encore de 1/15 à 1/50.

2. Composition concentrée selon la revendication 1, dans laquelle le co-tensioactif est choisi parmi la coco-amidopropylbétaïne, le coco-amidomonoéthanolamide et leurs mélanges.

3. Composition concentrée selon l'une quelconque des revendications précédentes, dans laquelle l'électrolyte inorganique est choisi parmi les chlorures métalliques, les sulfates métalliques et leurs mélanges.

4. Composition concentrée selon l'une quelconque des revendications précédentes, dans laquelle le rapport en poids du tensioactif anionique a) au co-tensioactif b) est de 3,5/1 à 9/1.

5. Composition concentrée selon l'une quelconque des revendications précédentes, qui comprend en outre de l'acide éthylènediaminetétraacétique.

6. Composition concentrée selon l'une quelconque des revendications précédentes, qui est exempte de silicone.

7. Procédé de préparation d'une composition pour usage final, le procédé comprenant l'étape de dilution d'une composition hydratable de l'une quelconque des revendications 1 à 6 avec de l'eau, avant utilisation.

8. Procédé selon la revendication 7, dans lequel le procédé comprend une étape d'application d'un cisaillement tel qu'un secouage ou une agitation au mélange de composition hydratable et d'eau pour engendrer la composition pour usage final en moins de 10 minutes.

9. Procédé selon la revendication 7 ou la revendication 8, dans lequel la composition est diluée dans un récipient.

10. Procédé selon la revendication 9, dans lequel le récipient est un emballage de recharge.

11. Procédé selon les revendications 7 à 10, dans lequel le rapport en poids de la composition de tensioactif concentrée hydratable à l'eau est de 1/1 à 1/6.

12. Procédé selon l'une quelconque des revendications 7 à 11, dans lequel la composition pour usage final est dans une phase isotrope.

13. Procédé selon l'une quelconque des revendications 7 à 12, dans lequel la composition pour usage final a une viscosité de 2 000 à 10 000 mPas⁻¹ (cps) quand elle est mesurée à 30 °C sur un dispositif Brookfield DV2T utilisant une broche RV-05 à 20 t/min pendant 60 secondes sur un support Helipath.
